(19) Europäisches Patentamt
European Patent Office
Office européen des brevets

(11) Publication number : **0 467 843 A2**

(12) **EUROPEAN PATENT APPLICATION**

(21) Application number : **91810550.3**

(51) Int. Cl.$^5$ : **C09K 15/14**

(22) Date of filing : **10.07.91**

(30) Priority : **19.07.90 US 555288**

(43) Date of publication of application :
**22.01.92 Bulletin 92/04**

(84) Designated Contracting States :
**BE DE ES FR GB IT NL**

(71) Applicant : **CIBA-GEIGY AG**
**Klybeckstrasse 141**
**CH-4002 Basel (CH)**

(72) Inventor : **Gatechair, Leslie R.**
**Rural Route 2, Box 60**
**Katonah, NY 10536 (US)**
Inventor : **Hyun, James**
**6 Kevin Drive**
**Danbury, CT 06811 (US)**

(54) **Prevention of oxidation in petrochemical extraction solvents.**

(57) A wide variety of hindered phenolic compounds are effective as antioxidant stabilizers to prevent the oxidation of liquid hydrocarbons and of oxygenated compounds, such as sulfolane or tetraethylene glycol, used as extraction solvents in the petroleum industry.

EP 0 467 843 A2

This invention pertains to a method of preventing the oxidation of liquid hydrocarbons and of oxygenated solvents used in the petroleum industry by the use of hindered phenolic compounds.

Organic compounds including liquid hydrocarbons and various oxygenated solvents are subject to oxidative and/or thermal degradation. The results of such unwanted oxidative reactions may be the loss of desirable properties, formation of color and/or odors and the presence of unwanted and corrosive by-products. A variety of liquid media are subject to such unwanted oxidation including saturated hydrocarbons, especially if they contain branched chains having tertiary carbon atoms.

T. Wheeler, Handbook of Petroleum Refinery Processes, R. A. Meyers, Edit. McGraw-Hill, New York, 1968, teaches the use of sulfolane to aid in the separation of $C_6$-$C_9$ aromatic hydrocarbons from aliphatic hydrocarbons having a similar boiling range, based on the selective solubility of the aromatic hydrocarbons in the sulfolane.

Somekh and Friedlander (Hydrocarbon Processing, 1969, pp. 127- 130 and Refining Petroleum for Chemicals, 1970, pp. 228-241) teach that glycols are effective solvents for extraction of aromatics from gasoline fractions during refining of petroleum. It is stated that small changes in solubility of aromatics or aliphatics in the separation or extraction solvent can have a dramatic effect on the efficiency of the entire process. Such changes can occur by adding a cosolvent such as water or by contamination of the solvent with heavy residues carried with the feedstock which effect the polarity of the solution. In addition, polarity can be affected by oxidation or hydrolysis of the solvent or rafinate resulting in a change in the partitioning of the aromatics and aliphatics in the extraction solvents.

Other saturated organic compounds, such as ethers, i.e. diethyl ether, tetraethylene glycol, are also subject to oxidation with the formation of peroxides which may result in fires and explosions. Such chemicals require special handling.

Some organic materials are solid at ambient temperature, but liquefy at slightly higher temperatures. Hydrocarbon waxes exemplify such materials which are subject to oxidation in the liquid state. Furfural is used to dissolve and remove paraffinic waxes from refinery streams.

In other cases, the organic material may be a solid which is dissolved in a solvent where it can be subject to oxidation.

In each of the above scenarios, the instant hindered phenolic compounds provide antioxidant stabilization protection to the organic material subject to oxidative or thermal degradation.

It is surmised that the instant hindered phenolic compounds act to prevent oxidation at carbon sites in the organic molecules having labile hydrogen atoms which are amenable to abstraction. Following abstraction of the hydrogen, oxygen may react with the site. Thus, an alkane is converted to peroxide, hydroperoxide or indirectly to an alcohol while an aldehyde may be converted to an acid, etc. Such reactions are free radical in nature and the addition of a free radical chain stopping agent acts to inhibit the chain propagation and to protect the site from oxidation.

United States Patent Nos. 3,644,278 and 3,778,464 describe the use of substituted hydroxylamine antioxidants in hydrocarbon fuels, kerosene, oils, paraffin, animal and vegetable oils. These patents also teach the use of the oxygen uptake method for screening the relative activity of antioxidants on various organic substrates dissolved in chlorobenzene. Typical substrates include tetralin, cyclohexane, lard, mineral oil and polymeric substances. No hindered phenolic compounds are disclosed in these references.

There still remains a need for effective stabilizers for materials as described above.

The instant invention pertains to a process for preventing the oxidation of a liquid organic substrate subject to oxidative or thermal degradation at temperatures below 200°C, which process comprises incorporating into said substrate a compound containing a moiety of formula I

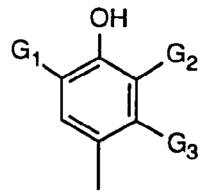

(I)

wherein

$G_1$ and $G_2$ are independently alkyl of 1 to 18 carbon atoms, alkenyl of 3 to 18 carbon atoms, cycloalkyl of 5 to 12 carbon atoms or phenylalkyl of 7 to 15 carbon atoms, and $G_3$ is hydrogen or alkyl of 1 to 4 carbon atoms.

Preferably $G_1$ and $G_2$ are independently alkyl of 1 to 8 carbon atoms, and $G_3$ is hydrogen.

Most preferably $G_1$ and $G_2$ are the same and are branched alkyl of 3 to 8 carbon atoms, especially tert-butyl.

Examples for compounds containing a moiety of formula I are compounds of formulae A, B, C or D

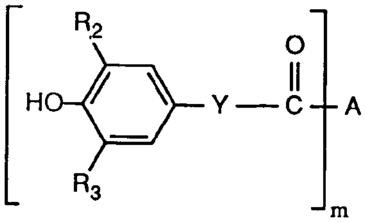

(A)

in which Y is a direct bond, methylene, ethylene or a

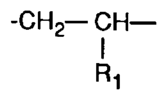

group and $R_1$ is $C_1$-$C_8$-alkyl, $R_2$ and $R_3$, independently of one another, are $C_1$-$C_{18}$alkyl, $C_5$-$C_{12}$cycloalkyl, $C_1$-$C_4$-alkyl-substituted $C_5$-$C_7$cycloalkyl, phenyl or $C_7$-$C_{10}$phenylalkyl and $R_3$ additionally is hydrogen, m is an integer from 1 to 6, and, where m is 1, A is a monovalent radical of a hexose or a hexitol, a

$$-O-CH_2-C(CH_2OH)_3,\ -O-[(CH_2)_t-O]_v-(CH_2)_t-OR',$$

or

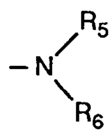

group in which t is an integer from 2 to 4 and v an integer from 2 to 20, R′ is hydrogen or $C_1$-$C_{12}$alkyl and $R_4$, $R_5$ and $R_6$ independently of one another, are hydrogen, $C_1$-$C_{24}$alkyl, $C_5$-$C_{12}$cycloalky, $C_1$-$C_4$alkyl-substituted $C_5$-$C_7$cycloalkyl or phenyl, or, where m is 2, A is a divalent radical of a hexose or a hexitol, a

$$-(O-CH_2)_2-C-(CH_2OH)_2,\ -O-X_1-O-,\ -NH-X_2-NH-,$$ 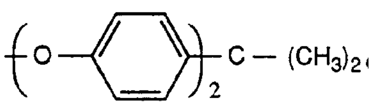

or

$$-O-[(CH_2)_t-O]_v$$

group in which

$X_1$ is

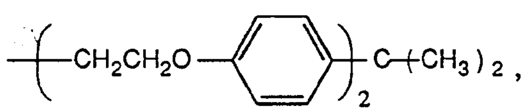

$C_2$-$C_{10}$alkylene, 3-thiapentane- 1,5-diyl, 2-butene-1,4-diyl or 2-butine-1,4-diyl, $X_2$ is $C_2$-$C_{10}$alkylene and t and v have the abovementioned meaning, or when m is 3, A is a trivalent radical of a hexose or a hexitol or a

$$-(O-CH_2)_3-C-R_7$$

group in which $R_7$ is hydrogen, $-CH_2OH$, $C_1$-$C_4$alkyl, ($C_1$-$C_{18}$-alkyl)amido or a

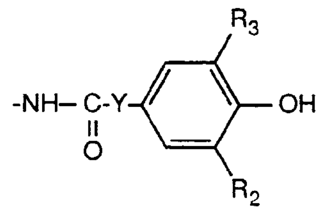

group and $R_3$, $R_2$ and Y have the abovementioned meaning, or, where m is 4, A is a tetravalent radical of a hexose or a hexitol,

$$-(O-CH_2)_4-C$$

or

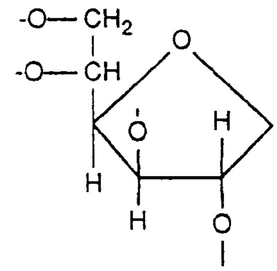

group, or, where m is 5, A is a pentavalent radical of a hexose or a hexitol and, where m is 6, A is a

$$-(O-CH_2)_3-C-CH_2-O-CH_2-C(CH_2-O)_3$$

group or a hexavalent radical of a hexitol;

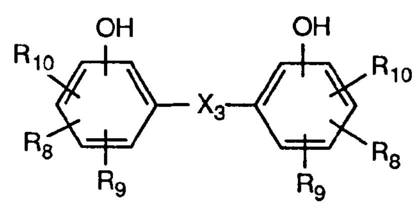

(B)

in which $R_{10}$, $R_8$ and $R_9$, independently of one another, are $C_1$-$C_{10}$cycloalkyl, $C_5$-$C_{12}$cycloalkyl, $C_1$-$C_4$alkyl-subsbtuted $C_5$-$C_7$cycloalkyl, phenyl or $C_7$-$C_{10}$phenylalkyl and $R_9$ is additionally hydrogen, $X_3$ is -S-, methylene, ethylidene, $C_3$-$C_6$alkylidene substituted in the 3-position by a

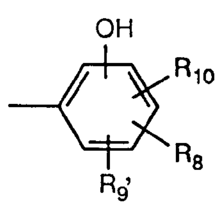

group
in which $R_{10}$ and $R_8$ have the abovementioned meanings and $R_9'$ is hydrogen, or $X_3$ furthermore is a

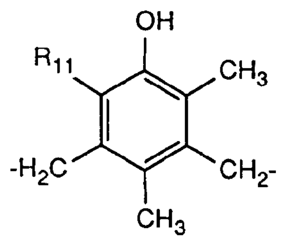

(B-1)

group in which $R_{11}$ is $C_1$-$C_{10}$alkyl or $C_5$-$C_{12}$cycloalkyl;

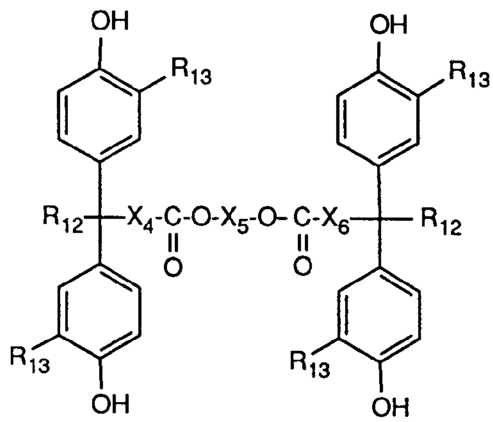

(C)

in which the radicals $R_{13}$, independently of one another, are $C_1$-$C_{10}$alkyl, $C_5$-$C_{12}$cycloalkyl or $C_1$-$C_4$alkyl-substituted $C_5$-$C_7$cycloalkyl, the radicals $R_{12}$ are methyl, $X_4$ and $X_6$, independently of one another, are methylene or ethylene and $X_5$ is $C_2$-$C_{10}$alkylene;

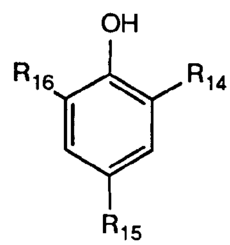

(D)

in which $R_{16}$ and $R_{14}$, independently of one another, are $C_1$-$C_{10}$alkyl, $C_5$-$C_{12}$cycloalkyl, $C_1$-$C_4$alkyl-substituted $C_5$-$C_7$cycloalkyl, phenyl or $C_7$-$C_{10}$phenylalkyl and $R_{14}$ is additionally hydrogen or $-CH_2SR_{17}$, $R_{15}$ is $C_1$-$C_{12}$alkyl or $-CH_2SR_{17}$, $R_{17}$ is $C_4$-$C_{18}$alkyl, phenyl, $C_7$-$C_{10}$phenylalkyl or a

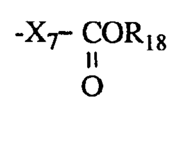

group in which $X_7$ is methylene or ethylene and $R_{18}$ is $C_1$-$C_{24}$alkyl.

Specifically preferred are compounds of formula II

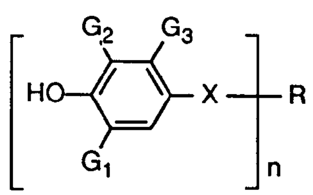

(II)

wherein $G_1$ and $G_2$ are independently alkyl of 1 to 18 carbon atoms, alkenyl of 3 to 18 carbon atoms, cycloalkyl of 5 to 12 carbon atoms or phenylalkyl of 7 to 15 carbon atoms,
$G_3$ is hydrogen or alkyl of 1 to 4 carbon atoms,
n is 1, 2, 3 or 4
X is a direct bond or a group $-(CH_2)_k-C(O)O-$,
wherein k is 0, 1 or 2,
when n is 1 R is hydrogen, straight or branched alkyl of 1 to 20 carbon atoms, said alkyl interrupted by one or more -O- or -S- atoms, alkenyl of 3 to 18 carbon atoms, cycloalkyl of 5 to 12 carbon atoms or phenylalkyl of 7 to 15 carbon atoms,
when n is 2, R is a direct bond, -S-, -S-S-, -SO-, $-SO_2-$, -O-, alkylene of 2 to 12 carbon atoms, said alkylene interrupted by 1 to 10 -O- or -S- atoms or R is alkylidene of 2 to 8 carbon atoms,
when n is 3, R is an alkanetriyl of 3 to 8 carbon atoms and
when n is 4, R is alkanetetrayl of 4 to 9 carbon atoms.

More specifically, the instant compounds are those of formula III

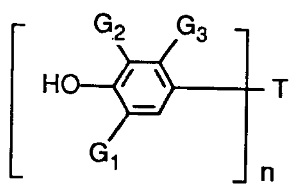

(III)

wherein
$G_1$, $G_2$ and $G_3$ are defined as above,
n is 1, 2 or 3,
T is an n-valent radical,
when n is 1, T is hydrogen, alkyl of 1 to 18 carbon atoms;
when n is 2, T is a direct bond, -S-, -S-S-, -SO-, $-SO_2-$, -O-, methylene or alkylidene of 2 to 8 carbon atoms; and
when n is 3, T is an alkanetriyl of 4 to 8 carbon atoms.

Preferably, when n is 1, T is hydrogen or alkyl of 1 to 4 carbon atoms, most preferably methyl.

Preferably, when n is 2, T is -S-, -S-S-, methylene or alkylidene of 2 to 4 carbon atoms.

Preferred compounds of formula III are 2,6-di-tert-butyl-4-methylphenol and 4,4'-dithiobis(2,6-di-tert-butylphenol).

Still other instant compounds are those of formula IV

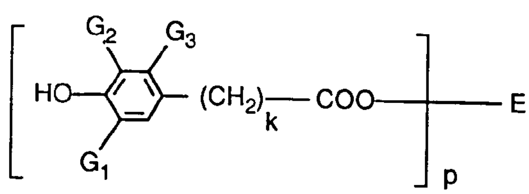

(IV)

wherein
$G_1$, $G_2$ and $G_3$ are defined as above,
k is 0, 1 or 2,
p is 1, 2, 3 or 4, and
E is a p-valent radical,
when p is 1, E is hydrogen, alkyl of 1 to 20 carbon atoms, said alkyl interrupted by one or more -O- or -S- atoms, alkenyl of 3 to 18 carbon atoms, cycloalkyl of 5 to 12 carbon atoms or phenylalkyl of 7 to 15 carbon atoms,
when p is 2, E is alkylene of 2 to 12 carbon atoms, said alkylene interrupted by one or more -S- atoms or $-CH_2CH_2(OCH_2CH_2)_q-$ where q is 1 to 10,
when p is 3, E is alkanetriyl of 3 to 8 carbon atoms, and
when p is 4, E is alkanetetrayl of 4 to 9 carbon atoms.

Preferably, k is 2.

Preferably, when p is 1, E is hydrogen or alkyl of 1 to 18 carbon atoms.

Preferably, when p is 2, E is alkylene of 4 to 8 carbon atoms or 3-thiapentamethylene.

Preferably, when p is 4, E is neopentanetetrayl.

Preferred compounds of formula IV are thiodiethylene bis(3,5-di-tert-butyl-4-hydroxyhydrocinnamate), 1,6-hexamethylene bis(3,5-di-tert-butyl-4-hydroxyhydrocinnamate), 3,5-di-tert-butyl-4-hydroxyhydrocinnamic acid, neopentanetetrayl tetrakis(3,5-di-tert-butyl-4-hydroxyhydrocinnamate), n-octadecyl 3,5-di-tert-butyl-4-hydroxyhydrocinnamate or 3,6-dioxaoctamethylene bis(3,5-di-tert-butyl-4-hydroxyhydrocinnamate).

The compounds of formulae I-IV are known and can be prepared by methods known per se.

The stabilizers of formulae I-IV usually are added in amounts that vary from 0.0001 to 0. 1 % by weight based on the weight of the organic liquid substrate being stabilized. Preferably the amount of the instant compound is 10 to 500 ppm by weight of the organic liquid being stabilized.

The preferred liquid substrates which are stabilized from oxidation by the instant compounds are light hydrocarbons used in liquid reprographic toners. The use of the instant compounds prevents the formation of odoriferous oxidabon products during storage and use under ambient or slightly elevated temperatures.

The prevention of oxidation and of peroxide formation in ethers, such as diethyl ether, and the like is another preferred embodiment of the instant invention. Preferably the substrate is an organic liquid subject to oxidative or thermal degradation at temperatures below 200°C selected from the group consisting of saturated aliphatic or cycloaliphatic hydrocarbons, oligomeric lower alkylene glycols, aliphatic aldehydes, sulfolane, N-methyl-2-pyrrolidone, lower alkyl nitriles, liquid butadiene rubber and furfural.

More preferably the substrate is selected from the group consisting of isooctane, heptane, cyclohexane, tetralin, squalene, polyethylene glycol, polypropylene glycol, polybutylene glycol, sulfolane, N-methyl-2-pyrrolidone, acetonitrile, liquid butadiene rubber and furfural.

Still more preferably, the substrate is tetraethylene glycol, sulfolane, N-methyl-2-pyrrolidone, acetonitrile or furfural.

It is also understood that any of the above-named extraction solvents may contain up to 5% by weight of water.

Additionally, mixtures of N-methyl-2-pyrrolidone with di-, tri-, tetra- of poly-ethylene glycol in the range of 10: 1 to 1: 10 with or without up to 5% by weight of water are also extraction solvents within the purvue of this invention.

However, the most preferred embodiment of the instant invention is the prevention of oxidation of selective extraction solvents used at temperatures of 100 to 150°C in the refining and separation of petroleum refinery streams. In practice the instant stabilizers can be added to the recycled extraction solvent or to the feed stream at a level of 10 ppb to 500 ppm as a makeup additive to maintain the desired concentration of said stabilizer in the extraction solvent. Typical extraction solvents are for example the ethylene, propylene or butylene glycol ethers, such as tetraethylene glycol, used in the UDEX process or sulfolane used in a similar process. Most preferably the substrate is the extraction solvent tetraethylene glycol or sulfolane.

The following examples are presented for the purpose of illustration only and are not to be construed to limit the nature or scope of the instant invention in any manner whatsoever.

Example 1: Oxidation of Selected Organic Compounds

A 1 molar solution of an organic compound selected from the group consisting of tetralin, lauryl aldehyde, polyethylene glycol of molecular weight 600 (PEG), squalene, a liquid butadiene rubber (PBD), isooctane and heptane in chlorobenzene is heated at 60°C in the presence of 0.03 mole of 2,2′-azobis(isobutyronitrile), AIBN, free radical initiator, and 0.002 mole of antioxidant stabilizer under an initial 1 atmosphere of oxygen. The rate of oxygen uptake in this AIBN initiated oxidation and the time in hours needed for 1.0 mmol or for 0.1 mmol of oxygen to be consumed are measured. A low rate of oxygen uptake and a longer time for consumption of the oxygen indicate a more effective antioxidant test stabilizer. The drop in oxygen pressure is a measure of the amount and rate of oxygen uptake.

The results are given on table 1 below.

Table 1

| | | Property*** | | | |
|---|---|---|---|---|---|
| Additive* | Substrate** | OCL-I | OCL-F | Time | Induction |
| None | tetralin | 10.0 | 10.0 | 3.7 | none |
| BHT | tetralin | 1.0 | 1.0 | 6.8 | 3.0 |
| Compound A | tetralin | 1.6 | 1.6 | 3.0# | none |
| None | lauryl A | 105 | 105 | 0.5 | none |
| BHT | lauryl A | 60 | 60 | 0.7 | none |
| Compound A | lauryl A | 46 | 46 | 0.6 | none |
| None | PBD | 6.8 | 6.8 | 5.6 | none |
| BHT | PBD | 1.5 | 1.5 | 8.3 | 3.0 |
| Compound A | PBD | 1.8 | 6.0 | 11.0 | 5.0 |
| None | PEG | 3.0 | 3.0 | 15.0 | none |
| BHT | PEG | 0.5 | 3.3 | 18.0 | 3.0 |
| Compound A | PEG | 1.0 | 2.7 | 3.6# | 2.0 |
| None | squalene | 17.0 | 17.0 | 2.1 | none |
| BHT | squalene | 3.2 | 14.0 | 5.4 | 3.4 |
| Compound A | squalene | 3.9 | 11.0 | 6.9 | 4.8 |
| None | isooctane | 0.5 | 0.5 | 7.7# | none |
| BHT | isooctane | 0.0 | 0.7 | 9.9# | 3.1 |
| Compound A | isooctane | 0.7 | 0.7 | 6.5# | none |

*BHT is 2,6-di-tert-butyl-4-methylphenol.

Compound A is 4,4'-dithiobis(2,6-di-tert-butylphenol).

**lauryl A is lauryl aldehyde.

PBD is liquid butadiene rubber.

PEG is polyethylene glycol of molecular weight of 600.

***OCL-I is Initial Oxidative Chain Length which is mols of oxygen consumed per moles of free radical generated.

OCL-F is Final Oxidative Chain Length which is the mols of oxygen consumed after failure per moles of free radical generated.

Time is in hours required to consume 1.0 mmol of oxygen.

# is time in hours to consume 0.1 mmol of oxygen.

Induction is the period in hours till rapid oxygen consumption begins.

Example 2: Oxidation of Tetraethylene Glycol

A 1 molar solution of tetraethylene glycol in chlorobenzene is heated at 60°C in the presence of 0.03 mole of 2,2'-azobis(isobutyronitrile), AIBN, and 0.002 mole of test stabilizer under an initial 1 atmosphere of oxygen. The rate of oxygen uptake in this AIBN initiated oxidation and final amount of oxygen consumed after 20 hours are measured. A low rate of oxygen uptake and a low total amount of oxygen consumed indicate a more effective antioxidant test stabilizer. The drop in oxygen pressure is a measure of the amount and rate of oxygen uptake.

The results are given in table 2

Table 2

| Test Stabilizer* | Initial Rate of Oxygen Consumption (mmol/hour) | Total Oxygen Consumption after 20 hours (mmol) |
|---|---|---|
| Blank | 0.09 | 1.47 |
| BHT | 0.03 | 1.20 |

*BHT is 2,6-di-tert-butyl-4-methylphenol.

Suppresion of oxidation is indicated both by the lower initial rate of oxidation and by the lower mmoles of oxygen consumed after 20 hours in the presence of the phenolic antioxidant BHT.

Example 3: Oxidation of Sulfolane

Following the procedure of Example 2, sulfolane (tetramethylene sulfone) is oxidized in the absence and in the presence of a phenolic antioxidant. The results are summarized on table 3

Table 3

| Test Stabilizer* | Initial Rate of Oxygen Consumption (mmol/hour) | Total Oxygen Consumption after 20 hours (mmol) |
|---|---|---|
| Blank | 0.02 | 0.18 |
| BHT | 0.007 | 0.17 |

*BHT is 2,6-di-tert-butyl-4-methylphenol.

Suppresion of oxidation is indicated both by the lower initial rate of oxidation and by lower mmoles of oxygen consumed after 20 hours in the presence of a phenolic antioxidant.

Example 4: Oxidation of Tetraethylene Glycol

Following the procedure of Example 2, the time in hours before the rapid consumption of oxygen begins is measured. This "induction" period is a measure of the effectiveness of the phenolic antioxidant in preventing the onset of rapid oxidation of the tetraethylene glycol substrate. The results are shown on table 4

Table 4

| Additive* | Induction Period (in hours) |
|---|---|
| Blank | 0.0 |
| BHT | 2.1 |
| Compound A | 2.0 |

*BHT is 2,6-di-tert-butyl-4-methylphenol.
Compound A is 4,4'-dithiobis(2,6-di-tert-butylphenol).

Example 5: Oxidation of Sulfolane

It is believed that the oxidation of the extraction solvent sulfolane (tetramethylene sulfone) results in the formation of acidic by-products which are probably complex blends of sulfuric, sulfurous and organosulfonic acids. The formation of such acidic products is clearly undesirable as it results in corrosion of processing equipment and the loss of sensitivity of the extraction solvent to selectively extract the petroleum refinery process stream. To measure the effectiveness of various additives on preventing the formation of acidic by-products in the sulfolane process, 50 ml of sulfolane containing 1.5% by weight of water is added to a 250 ml round bottomed flask topped with a condenser, bubbling inlet tube and a stopper. The contents of the flask are heated to 180°C while air is bubbled into the sulfolane at 125 ml/minute. The effluent air carrying any volatile oxidation products is in turn bubbled through 200 ml of water in a trap. After three hours of heating, aliquots from both the sulfolane and the water trap are titrated to determine non-volatile and volatile acid generated by the oxidation process. The resulting acidity is calculated in micromoles (μm) per gram of sulfolane tested. The results are shown in table 5.

Table 5

| Additive*(ppm) | Non-volatile Acidity (um/g) | Volatile Acidity (um/g) | Total Acidity (um/g) |
|---|---|---|---|
| None | 7.3 | 7.1 | 14.4 |
| Compound B (100) | 3.3 | 2.8 | 6.1 |
| Compound C (100) | 3.2 | 1.6 | 4.8 |
| Compound C (700) | 1.7 | 1.0 | 2.7 |

*Compound B is thiodiethylene bis(3,5-di-tert-butyl-4-hydroxyhydrocinnamate).

Compound C is 1,6-hexamethylene bis(3,5-di-tert-butyl-4-hydroxyhydrocinnamate).

This example demonstrates the reduced formation of acidic products of oxidation as prevented by the presence of low levels of a phenolic antioxidant.

Additionally, the usual formation of brown color and insoluble tar during the oxidation process is greatly suppressed by the presence of the phenolic antioxidant. Tar formation is a major problem encountered in aromatic compound separation processes.

Example 6: Retention of Phenolic Anboxidant in Sulfolane

Sulfolane is used to extract aromatic compounds from complex petroleum refinery process streams. Once extracted, the aromatic compounds are stripped from the sulfolane extraction solvent which is then recycled for additional use as an extraction solvent. It is clearly desirable that the phenolic antioxidant, meant to stabilize the sulfolane from oxidation, remain with the sulfolane during the stripping operation in which the extracted aromatic compounds are isolated from the extraction solvent.

To determine whether the phenolic antioxidant remains with the sulfolane during said stripping operation, to a solution of 50 g of sulfolane, having a boiling point of 285°C, and 50 g of mixed xylenes, having a boiling range of 137- 144°C is added 0. 1% by weight of the phenolic antioxidant being tested. The mixture is heated at temperatures up to 200°C to remove the xylenes by distillation. The xylene distillate is analyzed by gas chromatography to determine the amount of phenolic antioxidant present in the distillate.

The remaining antioxidant remains in the sulfolane.

Results are given on table 6.

Table 6

| Additive* | % Retained in Sulfolane |
|---|---|
| BHT | 98.5 |
| Compound C | >99.9 |
| Compound D | >99.9 |
| Compound E | >99.9 |

*BHT is 2,6-di-tert-butyl-4-methylphenol.

Compound C is 1,6-hexamethylene bis(3,5-di-tert-butyl-4-hydroxyhydrocinnamate).

Compound D is 3,5-di-tert-butyl-4-hydroxyhydrocinnamic acid.

Compound E is 3,6-dioxaoctamethylene bis(3,5-di-tert-butyl-4-hydroxyhydrocinnamate).

Example 7

When the procedure of Example 6 is repeated after substituting tetraethylene glycol for sulfolane, the results given in table 7 are obtained.

Table 7

| Additive* | % Retained in Tetraethylene Glycol |
|---|---|
| Compound B | > 99.9 |
| Compound C | > 99.9 |
| Compound F | > 99.9 |
| Compound G | > 99.9 |

*Compound B is thiodiethylene bis(3,5-di-tert-butyl-4-hydroxyhydrocinnamate).
Compound C is 1,6-hexamethylene bis(3,5-di-tert-butyl-4-hydroxyhydrocinnamate).

Compound F is neopentanetetrayl tetrakis(3,5-di-tert-butyl-4-hydroxyhydrocinnamate).

Compound G is n-octadecyl 3,5-di-tert-butyl-4-hydroxyhydrocinnamate.

Example 8

During a counter current extraction process, it is desirable that the phenolic antioxidant stabilizers remain in the aromatic compound/sulfolane phase. To measure this tendency, a mixture of 60 g of sulfolane, 600 mg of water, 300 mg of toluene, 10 g of heptane and 50 mg of a test additive is prepared. The mixture is shaken for one minute and the phases allowed to separate, The heptane layer floats on the heavier toluene/sulfolane layer. The heptane layer is separated off and then analyzed by liquid chromatography for any additive content. The partition coefficient is calculated to determine the tendency of the test additive to migrate to the heptane (H) or sulfolane (S) layer. A low H:S coefficient is desirable and indicates that the phenolic antioxidant prefers to remain in the more polar sulfolane layer. Similar results are also obtained with polar tetraethylene glycol. Results are given in table 8.

Table 8

| Additive* | Heptane: Sulfolane Partition Coefficient | Heptane: Tetraethylene glycol Partition Coefficient |
|---|---|---|
| BHT | 5.0:1 | 11.5:1 |
| Compound C | 1.1:1 | 22:1 |
| Compound D | <0.001:1 | <0.001:1 |
| Compound E | <0.001:1 | <0.001:1 |

*BHT is 2,6-di-tert-butyl-4-methylphenol.

Compound C is 1,6-hexamethylene bis(3,5-di-tert-butyl-4-hydroxyhydrocinnamate).

Compound D is 3,5-di-tert-butyl-4-hydroxyhydrocinnamic acid.

Compound E is 3,6-dioxaoctamethylene bis(3,5-di-tert-butyl-4-hydroxyhydrocinnamate).

## Claims

1. A process for preventing the oxidation of a liquid organic substrate subject to oxidative or thermal degradation at temperatures below 200°C, which process comprises incorporating into said substrate a compound containing a moiety of formula I

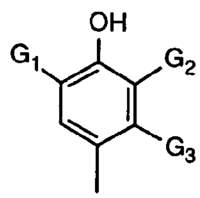

(I)

wherein $G_1$ and $G_2$ are independently alkyl of 1 to 18 carbon atoms, alkenyl of 3 to 18 carbon atoms, cycloalkyl of 5 to 12 carbon atoms or phenylalkyl of 7 to 15 carbon atoms, and
$G_3$ is hydrogen or alkyl of 1 to 4 carbon atoms.

2. A process according to claim 1 wherein the moiety of formula I, $G_1$ and $G_2$ are independently alkyl of 1 to 8 carbon atoms, and $G_3$ is hydrogen.

3. A process according to claim 2 wherein $G_1$ and $G_2$ are the same and are branched alkyl of 3 to 8 carbon atoms, especially tert-butyl.

4. A process accoding to claim 1, wherein the compound is a compound of formula II

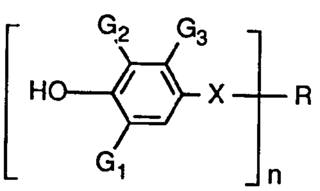

(II)

wherein $G_1$ and $G_2$ are independently alkyl of 1 to 18 carbon atoms, alkenyl of 3 to 18 carbon atoms, cycloalkyl of 5 to 12 carbon atoms or phenylalkyl of 7 to 15 carbon atoms,

$G_3$ is hydrogen or alkyl of 1 to 4 carbon atoms,
n is 1, 2, 3 or 4
X is a direct bond or a group $-(CH_2)_k-C(O)O-$,
wherein k is 0, 1 or 2,
when n is 1 R is hydrogen, straight or branched alkyl of 1 to 20 carbon atoms, said alkyl interrupted by one or more -O- or -S- atoms, alkenyl of 3 to 18 carbon atoms, cycloalkyl of 5 to 12 carbon atoms or phenylalkyl of 7 to 15 carbon atoms,
when n is 2, R is a direct bond, -S-, -S-S-, -SO-, $-SO_2-$, -O-, alkylene of 2 to 12 carbon atoms, said alkylene interrupted by 1 to 10 -O- or -S- atoms or R is alkylidene of 2 to 8 carbon atoms,
when n is 3, R is an alkanetriyl of 3 to 8 carbon atoms and
when n is 4, R is alkanetetrayl of 4 to 9 carbon atoms.

5. A process according to claim 1 wherein the compound, having present the moiety of formula I, is of formula III

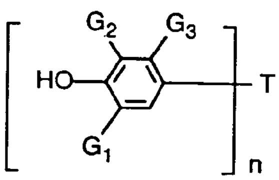

(III)

wherein $G_1$, $G_2$ and $G_3$ are defined as described in claim 1,
n is 1, 2 or 3,
T is an n-valent radical, and
when n is 1, T is hydrogen, alkyl of 1 to 18 carbon atoms;
when n is 2, T is a direct bond, -S-, -S-S-, -SO-, $-SO_2-$, -O-, methylene or alkylidene of 2 to 8 carbon atoms; and
when n is 3, T is an alkanetriyl of 4 to 8 carbon atoms.

6. A process according to claim 5 wherein, when n is 1, T is hydrogen or alkyl of 1 to 4 carbon atoms, especially methyl.

7. A process according to claim 5 wherein, when n is 2, T is -S-, -S-S-, methylene or alkylidene of 2 to 4 carbon atoms.

8. A process according to claim 1 wherein the compound, having present the moiety of formula I, is of formula IV

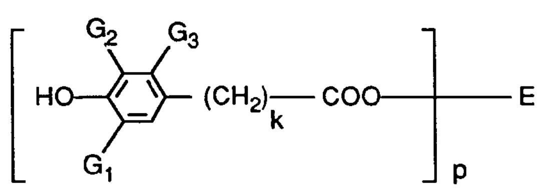

(IV)

wherein $G_1$, $G_2$ and $G_3$ are defined as described in claim 1,
k is 0, 1 or 2,
p is 1, 2, 3 or 4,
E is a p-valent radical, and
when p is 1, E is hydrogen, alkyl of 1 to 20 carbon atoms, said alkyl interrupted by one or more -O- or -S- atoms, alkenyl of 3 to 18 carbon atoms, cycloalkyl of 5 to 12 carbon atoms or phenylalkyl of 7 to 15 carbon atoms,
when p is 2, E is alkylene of 2 to 12 carbon atoms, said alkylene interrupted by one or more -S- atoms or $-CH_2CH_2(OCH_2CH_2)_q-$ where q is 1 to 10,
when p is 3, E is alkanetriyl of 3 to 8 carbon atoms, and
when p is 4, E is alkanetetrayl of 4 to 9 carbon atoms.

**9.** A process according to claim 8 wherein k is 2.

**10.** A process according to claim 8 wherein, when p is 1, E is hydrogen or alkyl of 1 to 18 carbon atoms.

**11.** A process according to claim 8 wherein, when p is 2, E is alkylene of 4 to 8 carbon atoms or 3-thiapentamethylene.

**12.** A process according to claim 8 wherein, when p is 4, E is neopentanetetrayl.

**13.** A process according to claim 1 wherein the substrate is an organic liquid subject to oxidative or thermal degradation at temperatures below 200°C selected from the group consisting of saturated aliphatic or cycloaliphatic hydrocarbons, oligomeric lower alkylene glycols, aliphatic aldehydes, sulfolane, N-methyl-2-pyrrolidone, lower alkyl nitriles, liquid butadiene rubber and furfural.

**14.** A process according to claim 13 wherein the substrate is selected from the group consisting of isooctane, heptane, cyclohexane, tetralin, squalene, polyethylene glycol, polypropylene glycol, polybutylene glycol, sulfolane, N-methyl-2-pyrrolidone, acetonitrile, liquid butadiene rubber and furfural.

**15.** A process according to claim 13 wherein the substrate is tetraethylene glycol, sulfolane, N-methyl-2-pyrrolidone, acetonitrile or furfural, especially tetraethylene glycol or sulfolane.